# EUROPEAN PATENT SPECIFICATION

(11) **EP 1 601 388 B1**
(45) Date of publication and mention of the grant of the patent: **27.04.2011**
(21) Application number: 04716655.8
(22) Date of filing: 03.03.2004
(51) Int. Cl.: A61L 26/00

(54) **HYDROCOLLOID MATERIALS FOR USE IN WOUND HEALING**
HYDROKOLLOID-MATERIALIEN ZUR VERWENDUNG BEI DER WUNDHEILUNG
MATERIAUX HYDROCOLLOIDES DESTINES A ETRE UTILISES DANS LA CICATRISATION DES BLESSURES

(30) Priority: 10.03.2003 GB 0305454; 31.03.2003 US 458383 P
(43) Date of publication of application: 07.12.2005
(73) Proprietor: Johnson & Johnson Medical Ltd., Edinburgh EH2 4NH (GB)
(72) Inventor: SILCOCK, Derek, Walter, Skipton BD23 1QP (GB); DELBONO, Michelle, Barnoldswick BB18 5HJ (GB)
(74) Representative: James, Anthony Christopher W.P.
(86) International application number: PCT/GB2004/000892
(87) International publication number: WO 2004/080500

(56) References cited:
- EP-A- 0 815 879
- WO-A-01/24841
- WO-A-98/00180
- WO-A-2004/026200
- US-A- 5 696 101

## Description

The present invention relates to low-moisture gel compositions for use in wound dressings. The invention also relates to the manufacture of such low-moisture gel compositions, and to methods of wound treatment using such compositions.

EP-A-0918548 describes the use of oxidized celluloses, such as oxidized regenerated cellulose (ORC), for the treatment of wounds, in particular chronic wounds. Compositions are described that comprise milled ORC fibers dispersed in a 3% sodium carboxymethyl cellulose (NaCMC) aqueous hydrogel. Another example describes a composite film comprising ORC fibers dispersed in a plasticized collagen matrix. In other embodiments, the ORC fibers are dispersed in an aqueous collagen slurry and the mixture is freeze-dried to produce a collagen/ORC sponge having especially good properties for the treatment of chronic wounds. Such freeze-dried sponges are commercially available from Johnson & Johnson Medical Limited under the registered trade mark PROMOGRAN.

EP-A-0562862, EP-A-0562863 and EP-A-0562864 describe freeze-dried collagen sponges optionally having macroscopic substructures made of ORC embedded in the sponge. One embodiment consists of an ORC fabric laminated to a layer of collagen sponge and a layer of plasticized collagen film.

EP-A-0636378 describes medicated collagen films for use in the treatment of periodontal disease. The medicated collagen films may contain dispersed ORC fibers. The collagen matrix may contain up to 20% by weight, based on the weight of the composite of a plasticizer.

A need remains for further wound dressing materials containing ORC that can provide advantages over ORC/CMC gels or collagen/ORC sponges in terms of cost, stability, ease of manufacture and/or other properties.

The present inventors have found that a stable, conformable wound dressing material can be made by dispersing solid ORC in an aqueous gel, followed by drying the gel to produce a stable, flexible hydrogel matrix material containing dispersed ORC.

EP-A-0815879 describes medical devices and wound dressings comprising an oxidized carboxymethylcellulose (CMC). The oxidized CMC has the properties of being non-adherent and water-soluble. In certain embodiments, an oxidized regenerated cellulose (ORC) cloth is coated with the oxidized carboxymethylcellulose (CMC).

In a first aspect the present invention provides a wound dressing material in the form of a plasticised solid sheet or layer of a low-moisture hydrogel matrix having oxidised regenerated cellulose distributed therein, wherein said material comprises from 1% to 50% by weight of water, from 1 % to 80% by weight of one or more hydrocolloids, from 10% to 80% by weight of plasticiser, and from 1% to 40% by weight of oxidised regenerated cellulose.

The term "hydrogel matrix" refers to a continuous solid phase having the oxidized cellulose dissolved or embedded therein. The Hydrogel matrix is not a freeze-dried sponge material, and preferably it has an uncompressed density of at least about 0.2g/cm³, more preferably at least about 0.4g/cm³.

The hydrogel matrix comprises one or more gel-forming hydrocolloids. The term "gel-forming hydrocolloid" refers to a polymeric material that absorbs water, for example from wound fluid, to form a coherent gel under physiological conditions of temperature and pH. Preferably, the hydrocolloid absorbs at least 100%w/w, more preferably at least 300%w/w of water on immersion at 25°C for 24 hours. The matrix is preferably soluble in excess water, so as to release dispersed ORC into the wound in use. In other embodiments the matrix is water-swellable, but not water-soluble. In preferred embodiments of this type, the matrix may be formed of a hydrogel material that breaks down gradually *in vivo* so as to provide sustained release the oxidized cellulose into the wound. The term "hydrogel" in this context does not include gels or films comprising substantial amounts (e.g. more than 50% by weight) of gel-forming proteins or peptides such as collagen or gelatin. Preferably, the hydrogel material according to the present invention is substantially free of collagen and gelatin, and more preferably it is substantially free of other gel-forming proteins or peptides.

In certain embodiments the hydrogel matrix comprises a hydrocolloid material selected from the group consisting of modified celluloses, modified starches, alginates, plant gums, glycosaminoglycans, polyacrylates, polyurethanes, polymers of vinyl alcohols, vinyl esters, vinyl ethers and carboy vinyl monomers, meth(acrylic) acid, acrylamide, N-vinyl pyrrolidone, acylamidopropane sulfonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof.

In preferred embodiments the hydrocolloids comprise a naturally occurring or chemically modified polysaccharide, and preferably they consists essentially of one or more naturally occurring or chemically modified polysaccharides, or mixtures thereof. Suitable chemically modified polysaccharides are selected from the group consisting of carboxymethyl cellulose gels, hydroxyethyl cellulose gels, hydroxy propyl methyl cellulose gels, chitosan, low-methoxy pectins, cross-linked dextran and starch-acrylonitrile graft copolymer, starch sodium polyacrylate, and mixtures thereof. Suitable natural polysaccharides include alginic acid and its salts, pectins, galactomannans such as xanthan gum or guar gum locust bean gum, gum karaya, gum arabic, hyaluronic acid and its salts, starches, and mixtures thereof. Suitably synthetic hydrocolloids include high molecular weight polyethylene glycols and polypropylene glycols, polymers of methyl vinyl ether and maleic acid and derivatives; polyvinyl pyrrolidone, polyethylene glycols, polypropylene glycols, metal and/or ammonium salts of polyacrylic acid and/or its copolymers, metal or ammonium salts of polystyrene sulfonic acid, and mixtures thereof.

Especially preferred are compositions in which the hydrocolloids comprise, or consist essentially of, a carboxymethyl cellulose salt.

Preferably, the oxidized regenerated cellulose is a solid material forming a distinct solid phase embedded in the matrix. However, in certain embodiments the oxidized regenerated cellulose may be in the form of dissolved soluble fragments, as described in EP-A-0907664.

Preferably, the oxidized regenerated cellulose is in the form of fibers or fiber fragments, more preferably in the form of a woven or non-woven fabric, or discrete fibers or fiber fragments dispersed in the matrix. Preferably, the fibers or fiber fragments are randomly dispersed in the matrix.

The wound dressing material according to the present invention further comprises a plasticizer. Suitable plasticisers include glycerol, propylene glycol, polyethylene glycol, polypropylene glycol, sorbitol, other glycols and ether glycols such as mono- or diethers of polyalkylene glycol, mono- or diester polyalkylene glycols, polyethylene glycols glycolates, ethylene glycol, diethylene glycol, triethylene glycol, propylene glycol dipelargonate and polypropylene glycol glycerol, sorbitan esters, esters of citric and tartaric acid, imidazoline derived amphoteric surfactants, lactams, amides, polyamides, quaternary ammonium compounds, esters such phthalates, adipates, stearates, palmitates, sebacates, or myristates, and combinations thereof. diisopropyl adipate, phthalates and diethyl sebacate; hydrocarbons such as liquid paraffin; ethoxylated stearyl alcohol, glycerol esters, isopropyl myristate, isotridecyl myristate, ethyl laureate, N-methylpyrrolidone, ethyl oleate, oleic acid, isopropyl adipate, isopropyl palmitate, octyl palmitate, 1,3-butanediol and mixtures thereof. The wound dressing material comprises from 10% to 80% by weight of the plasticizer, preferably from 15% to 60% by weight of the plasticiser.

The hydrogel matrix is a low-moisture hydrogel material. It has been found that the low moisture content stabilizes the ORC in the matrix, and provides related advantages. The wound dressing material comprises from 1 % to 50% by weight of water, preferably from 5% to 40% by weight of water, more preferably from 10% to 30% by weight of water.

The wound dressing Material according to the present invention comprises from 1% to 40% by weight of the oxidized regenerated cellulose, preferably from 5% to 25% by weight.

The wound dressing material according to the present invention comprises from 1% to 80% by weight of the one or more hydrocolloids, preferably from 25% to 60% by weight.

Preferably, the wound dressing material according to the present invention further comprises one or more therapeutic agents for promoting or enhancing wound healing. The one or more therapeutic agents may be any substance suitable for the treatment of wounds, but does not include the hydrogel used to form the matrix or the oxidized cellulose itself, both of which may independently promote wound healing. In certain embodiments the therapeutic agents are selected from the group consisting of antiseptics, antibiotics, analgesics, steroids and growth factors. Preferred therapeutic agents are the antimicrobials, in particular antibiotics and antiseptics such as colloidal silver, silver sulfadiazine, povidone iodine, chlorhexidine, and mixtures thereof. Preferably, the wound dressing material according to the present invention comprises from 0.001% to 10% by weight of the one or more therapeutic substances, more preferably from 0.05% to 2% by weight.

In a second aspect, the present invention provides a wound dressing comprising a wound dressing material according to the present invention.

The material according to the present invention is in the form of a solid sheet (or layer) for application to a wound. Preferably, the sheet is from 0.1 to 2mm thick. Preferably, the sheet has a dry basis weight of from about 10 to about 1000g/m², more preferably from about 20 to about 200g/m², and most preferably from about 40 to about 100g/m². The sheet may be laminated to a solid polymer film, for example a perforated wound contacting film of ethylene methyl acrylate (EMA).

The sheet or layer of the material according to the present invention may be continuos or apertured. Typically, the apertures make up from about 0.1% to about 50% of the area of the sheet (preferably of the wound facing area of the sheet) before swelling, more typically from about 1% to about 30% of the area of the sheet before swelling, preferably from about 10% to about 25%, and more preferably from about 10% to about 20% of the area of the sheet before swelling.

It is an advantage of the present invention that the low moisture hydrogels have a conformable and slightly resilient feel that makes such sheets suitable for handling and application to a wound surface.

Preferably, the wound dressing comprises an absorbent layer and/or a backing layer in addition to the sheet of material according to the present invention, in which case the sheet is preferably the wound-facing top sheet of the dressing.

Preferably, the dressing further comprises a backing layer over the back face of the dressing sheet. The backing layer preferably provides a barrier to passage of microorganisms through the dressing and further preferably blocks the escape of wound fluid from the dressing. The backing layer may extend beyond at least one edge of the absorbent layer to provide an adhesive-coated margin adjacent to the said edge for adhering the dressing to a surface, such as to the skin of a patient adjacent to the wound being treated. An adhesive-coated margin may extend around all sides of the absorbent layer, so that the dressing is a so-called island dressing. However, it is not necessary for there to be any adhesive-coated margin.

Preferably, the backing layer is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-Cell. A suitable backing sheet material is the polyurethane film available under the (Registered Trade Mark ESTATE 5714F.

The adhesive (where present) layer should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Preferably, the adhesive layer extends outwardly from the absorbent layer and the envelope to form an adhesive-coated margin on the backing sheet around the adhesive layer as in a conventional island dressing.

The area of the optional absorbent layer is typically in the range of from 1cm² to 200cm², more preferably from 4cm² to 100cm².

The optional absorbent layer may be any of the layers conventionally used for absorbing wound fluids, serum or blood in the wound healing art, including gauzes, nonwoven fabrics, superabsorbents, hydrogels and mixtures thereof. Preferably, the absorbent layer comprises a layer of absorbent foam, such as an open celled hydrophilic polyurethane foam prepared in accordance with EP-A-0541391. In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1 mm to 4mm. The free (uncompressed) liquid absorbency measured for physiological saline may be in the range of 5 to 30 g/g at 25°.

Preferably, the wound dressing according to the invention is sterile and packaged in a microorganism-impermeable container.

In a third aspect, the present invention provides the use of a wound dressing material, according to the present invention, for the preparation of a dressing for use in the treatment of wounds.

Preferably, the wound is a chronic wound such as a venous ulcer, a diabetic ulcer or a decubitis ulcer.

In a further aspect, the present invention provides a method of making a wound dressing material according to the present invention, comprising the steps of: providing an aqueous gel; immersing or dispersing an oxidized cellulose in the aqueous hydrocolloid gel; followed by drying the gel to a moisture content of less than 50% by weight.

The aqueous gel preferably comprises at least about 90%w/w of water, more preferably at least about 95%w/w of water, The step of drying is preferably carried out under mild conditions. Preferably, the step of drying is applied without applying a vacuum, and preferably the step of drying is carried out at a temperature of from about 0 to about 100°C, more preferably from about 20º to about 80ºC, and most preferably at from about 35º to about 65ºC. The material may be shaped, for example by molding or extrusion, either before, after, or in the course of the drying step.

It will be appreciated that any features that are described as alternatives or preferred features in connection with any one of the above aspects of the invention are also likewise alternatives or preferred in relation to any other aspect of the invention.

Specific embodiments of the invention will now be described further, by way of example.

### Example 1

Milled ORC fibers prepared as described in EP-A-1153622 were dispersed at a concentration of 2.17% w/w in KY Jelly (registered trade mark). KY Jelly is a commercial hydrogel manufactured by Johnson & Johnson. The exact formulation is as follows, in percentages by weight:

| | |
|---|---|
| Propylene glycol | 3.750 |
| Glycerine | 11.250 |
| Monosodium Dihydrogen orthophosphate (buffer) | 0.875 |
| Disodium hydrogen orthophosphate (buffer) | 0.045 |
| Methyl butex (preservative) | 0.100 |
| Propyl ester (preservative) | 0.040 |
| Hydroxyethyl cellulose | 2.133 |
| EDTA | 0.021 |
| Water | 81.792 |

The resulting gel was spread in a petri dish to a depth of 5mm and dried in air immediately at 37°C for 48 hours. The resulting dried hydrogel layer was flexible, conformable, and slightly elastic. The ORC in the hydrogel matrix appeared to be completely stable for at least six weeks at 37°C in ambient atmosphere. That is to say, the ORC fibers when viewed under a microscope did not exhibit any swelling or dissolution.

### Example 2

The procedure of Example 1 was repeated, but with replacement of the KY jelly with INTRASITE (Registered Trade Mark) gel produced by Smith & Nephew Healthcare Ltd. This is an aqueous gel which contains 2.3% of a modified CMC and 20% of propylene glycol.

## Claims

1. A wound dressing material in the form of a plasticised solid sheet or layer of a low-moisture hydrogel matrix having oxidised regenerated cellulose distributed therein, wherein said material comprises from 1% to 50% by weight of water, from 1% to 80% by weight of one or more hydrocolloids, from 10% to 80% by weight of plasticiser, and from 1% to 40% by weight of oxidised regenerated cellulose.

2. A wound dressing material according to claim 1, wherein the oxidized regenerated cellulose is in the form of discrete fibers.

3. A wound dressing material according to any preceding claim, wherein the hydrogel matrix comprises a hydrogel selected from the group consisting of modified celluloses, modified starches, alginates, plant gums, gelatins, glycosaminoglycans, polyacrylates, polyurethanes, and mixtures thereof.

4. A wound dressing material according to claim 3, wherein the hydrogel is selected from the group consisting carboxymethyl cellulose salts, alginate salts, gelatins, hyaluronic acid and its salts, xanthan gum, guar gum, and mixtures thereof.

5. A wound dressing material according to any preceding claim, wherein the plasticizer is selected from the group consisting of glycerol, propylene glycol, polyethylene glycol, polypropylene glycol, sorbitol, other glycols and ether glycols such as mono- or diethers of polyalkylene glycol, mono- or diester polyalkylene glycols, polyethylene glycols glycolates, ethylene glycol, diethylene glycol, triethylene glycol, propylene glycol dipelargonate and polypropylene glycol glycerol, sorbitan esters, esters of citric and tartaric acid, imidazoline derived amphoteric surfactants, lactams, amides, polyamides, quaternary ammonium compounds, esters such phthalates, adipates, stearates, palmitates, sebacates, or myristates, and combinations thereof, diisopropyl adipate, phthalates and diethyl sebacate; hydrocarbons such as liquid paraffin; ethoxylated stearyl alcohol, glycerol esters, isopropyl myristate, isotridecyl myristate, ethyl laurate, N-methylpyrrolidone, ethyl oleate, oleic acid, isopropyl adipate, isopropyl palmitate, octyl palmitate, 1,3-butanediol and mixtures thereof.

6. A wound dressing material according to any preceding claim, wherein the wound dressing material comprises from 15% to 60% by weight of the plasticizer.

7. A wound dressing material according to any preceding claim, wherein the wound dressing material comprises from 5% to 40% by weight of water.

8. A wound dressing material according to any preceding claim, wherein the material comprises from 5% to 25% by weight of the oxidized regenerated cellulose.

9. A wound dressing material according to any preceding claim, wherein the material comprises from 25% to 60% by weight of the one or more hydrocolloids.

10. A wound dressing material according to any preceding claim, further comprising one or more therapeutic agents.

11. A wound dressing comprising a wound dressing material according to any of claims 1 to 10.

12. A wound dressing according to claim 11, which is sterile and packaged in a microorganism-impermeable container.

13. Use of a wound dressing material according to any of claims 1 to 10, for the preparation of a dressing for use in the treatment of wounds.

14. Use according to claim 13, wherein the wound is a chronic wound.

15. A method of making a wound dressing material according to any of claims 1 to 10, comprising the steps of:
providing an aqueous hydrocolloid gel;
immersing or dispersing an oxidized regenerated cellulose in the aqueous gel; followed by
drying the gel to a moisture content of less than 50% by weight.

## Patentansprüche

1. Wundabdeckmaterial in der Form einer plastifizierten festen Lage oder Schicht aus einer Hydrogelmatrix mit niedriger Feuchte mit darin verteilter oxidierter regenerierter Cellulose, wobei das Material von 1 bis 50 Gew.-% Wasser, von 1 bis 80 Gew.-% eines oder mehrerer Hydrokolloide, von 10 bis 80 Gew.-% Weichmacher und von 1 bis 40 Gew.-% oxidierte regenerierte Cellulose umfasst.

2. Wundabdeckmaterial nach Anspruch 1, wobei die oxidierte regenerierte Cellulose in der Form diskreter Fasern vorliegt.

3. Wundabdeckmaterial nach einem vorangehenden Anspruch, wobei die Hydrogelmatrix ein Hydrogel umfasst, das ausgewählt ist aus der Gruppe, bestehend aus modifizierten Cellulosen, modifizierten Stärken, Alginaten, Pflanzengummis, Gelatinen, Glykosaminoglykanen, Polyacrylaten, Polyurethanen und Mischungen davon.

4. Wundabdeckmaterial nach Anspruch 3, wobei das Hydrogel ausgewählt ist aus der Gruppe, bestehend aus Carboxymethylcellulosesalzen, Alginatsalzen, Gelatinen, Hyaluronsäure und ihren Salzen, Xanthangummi, Guargummi und Mischungen davon.

5. Wundabdeckmaterial nach einem vorangehenden Anspruch, wobei der Weichmacher ausgewählt ist aus der Gruppe, bestehend aus Glycerol, Propylenglykol, Polyethylenglykol, Polypropylenglykol, Sorbitol, anderen Glykolen und Etherglykolen, wie etwa Mono- oder Diethern von Polyalkylenglykol, Mono- oder Diesterpolyalkylenglykolen, Polyethylenglykolglykolaten, Ethylenglykol, Diethylenglykol, Triethylenglykol, Propylenglykoldipelargonat und Polypropylenglykolglycerol, Sorbitanestern, Estern von Citronen- und Weinsäure, von Imidazolin abgeleiteten amphoteren Tensiden, Lactamen, Amiden, Polyamiden, quartären Ammoniumverbindungen, Estern wie Phthalaten, Adipaten, Stearaten, Palmitaten, Sebacaten oder Myristaten, und Kombinationen davon, Düsopropyladipat, Phthalaten und Diethylsebacat; Kohlenwasserstoffen, wie etwa Flüssigparaffin; ethoxyliertem Stearylalkohol, Glycerolestern, Isopropylmyristat, Isotridecylmyristat, Ethyllaurat, N-Methylpyrrolidon, Ethyloleat, Ölsäure, Isopropyladipat, Isopropylpalmitat, Octylpalmitat, 1,3-Butandiol und Mischungen davon.

6. Wundabdeckmaterial nach einem vorangehenden Anspruch, wobei das Wundabdeckmaterial von 15 bis 60 Gew.-% des Weichmachers umfasst.

7. Wundabdeckmaterial nach einem vorangehenden Anspruch, wobei das Wundabdeckmaterial von 5 bis 40 Gew.-% Wasser umfasst.

8. Wundabdeckmaterial nach einem vorangehenden Anspruch, wobei das Material von 5 bis 25 Gew.-% der oxidierten regenerierten Cellulose umfasst.

9. Wundabdeckmaterial nach einem vorangehenden Anspruch, wobei das Material von 25 bis 60 Gew.-% des einen oder der mehreren Hydrokolloide umfasst.

10. Wundabdeckmaterial nach einem vorangehenden Anspruch, das weiter ein oder mehrere therapeutische Mittel umfasst.

11. Wundabdeckung, umfassend ein Wundabdeckmaterial nach einem der Ansprüche 1 bis 10.

12. Wundabdeckung nach Anspruch 11, die steril und in einem für Mikroorganismen undurchlässigen Behälter abgepackt ist.

13. Verwendung eines Wundabdeckmaterials nach einem der Ansprüche 1 bis 10 zur Herstellung einer Abdeckung zur Verwendung bei der Behandlung von Wunden.

14. Verwendung nach Anspruch 13, wobei die Wunde eine chronische Wunde ist.

15. Verfahren zur Herstellung eines Wundabdeckmaterials nach einem der Ansprüche 1 bis 10, welches die Schritte umfasst:
Bereitstellen eines wässrigen Hydrokolloidgels;
Eintauchen oder Dispergieren einer oxidierten regenerierten Cellulose im wässrigen Gel; gefolgt von
Trocknen des Gels bis zu einem Feuchtegehalt von weniger als 50 Gew.-%.

## Revendications

1. Matériau de pansement sous la forme d'une feuille ou couche solide plastifiée d'une matrice d'hydrogel à basse teneur en humidité dans laquelle est distribuée de la cellulose oxydée régénérée, où ledit matériau comprend de 1% à 50% en poids d'eau, de 1% à 80% en poids d'un ou de plusieurs hydrocolloïdes, de 10% à 80% en poids de plastifiant et de 1% à 40% en poids de cellulose oxydée régénérée.

2. Matériau de pansement selon la revendication 1, dans lequel la cellulose oxydée régénérée se présente sous la forme de fibres discrètes.

3. Matériau de pansement selon l'une quelconque des revendications précédentes, dans lequel la matrice d'hydrogel comprend un hydrogel sélectionné dans le groupe consistant en celluloses modifiées, amidons modifiés, alginates, gommes de plantes, gélatines, glycosaminoglycanes, polyacrylates, polyuréthanes et leurs mélanges.

4. Matériau de pansement selon la revendication 3, dans lequel l'hydrogel est sélectionné dans le groupe consistant en sels de carboxyméthyl cellulose, sels d'alginate, gélatines, acide hyaluronique et ses sels, gomme xanthique, gomme de guar et leurs mélanges.

5. Matériau de pansement selon l'une quelconque des revendications précédentes, dans laquelle le plastifiant est sélectionné dans le groupe consistant en glycérol, propylène glycol, polyéthylène glycol, polypropylène glycol, sorbitol, autres glycols et éther glycols comme des mono- ou diéthers de polyalkylène glycol, des mono- ou diesters de polyalkylène glycol, des glycolates de polyéthylène glycol, éthylène glycol, diéthylène glycol, triéthylène glycol, dipélargonate de propylène glycol et glycérol de polypropylène glycol, sorbitan esters, esters de l'acide citrique et tartarique, agents de surface amphotériques dérivés d'imidazoline, lactames, amides, polyamides, composés d'ammonium quaternaire, esters comme des phtalates, adipates, stéréates, palmitates, sébacates ou myristates et leurs combinaisons, diisopropyl adipate, phtalates et diéthyl sébacate; des hydrocarbures comme la paraffine liquide; le stéaryl alcool éthoxylé, les esters de glycérol, isopropyl myristate, isotridécyl myristate, éthyl laurate, N-méthylpyrrolidone, éthyl oléate, acide oléique, isopropyl adipate, isopropyl palmitate, octyl palmitate, 1,3-butanediol et leurs mélanges.

6. Matériau de pansement selon l'une quelconque des revendications précédentes, dans lequel le matériau de pansement comprend de 15% à 60% en poids de plastifiant.

7. Matériau de pansement selon l'une quelconque des revendications précédentes, dans lequel le matériau de pansement comprend de 5% à 40% en poids d'eau.

8. Matériau de pansement selon l'une quelconque des revendications précédentes, dans lequel le matériau comprend de 5% à 25% en poids de la cellulose oxydée régénérée.

9. Matériau de pansement selon l'une quelconque des revendications précédentes, dans lequel le matériau comprend de 25% à 60% en poids d'un ou de plusieurs hydrocolloïdes.

10. Matériau de pansement selon l'une quelconque des revendications précédentes, comprenant en outre un ou plusieurs agents thérapeutiques.

11. Pansement comprenant un matériau de pansement selon l'une quelconque des revendications 1 à 10.

12. Pansement selon la revendication 11, qui est stérile et emballé dans un contenant imperméable aux microorganismes.

13. Utilisation d'un matériau de pansement selon l'une quelconque des revendications 1 à 10 pour la préparation d'un pansement utilisé dans le traitement des plaies.

14. Utilisation selon la revendication 13, où la plaie est une plaie chronique.

15. Procédé de fabrication d'un matériau de pansement selon l'une quelconque des revendications 1 à 10, comprenant les étapes de:
réaliser un gel hydrocolloïde aqueux;
immerger ou disperser une cellulose oxydée régénérée dans le gel aqueux; suivi du séchage du gel à une teneur en humidité inférieure à 50% en poids.
